# EUROPEAN PATENT APPLICATION

(11) **EP 4 368 133 A1**
(43) Date of publication of application: **15.05.2024**
(21) Application number: 23209183.5
(22) Date of filing: 10.11.2023
(51) Int. Cl.: A61B 18/14, A61B 18/00

(54) **ELECTRODE CATHETER WITH CORRUGATED SUPPORT STRUCTURE**

(30) Priority: 11.11.2022 US 202263383445 P; 11.10.2023 US 202318485002
(71) Applicant: BIOSENSE WEBSTER (ISRAEL) LTD., 2066717 Yokneam (IL)
(72) Inventor: EBRAHIMI, Babak, Irvine, 92618 (US); VAN NIEKERK, Pieter Emmelius, Irvine, 92618 (US); RODRIGUEZ SOTO, Juan, Irvine, 92618 (US)
(74) Representative: Carpmaels & Ransford LLP

(57) **Abstract**

An effector having a support frame with one or more corrugated struts is presented herein. Corrugation of a strut increases lateral stiffness of the strut and decreases axial bending stiffness of the strut compared to a linear, non-corrugated strut of similar thickness. Geometry of corrugations can be selected to provide conformal contact of the end effector to tissue while maintaining a desired spatial relationship between electrodes of the end effector. The geometry that can be selected can include amplitude of undulations, wavelength of undulations, and placement of a particular geometry within the end effector.

## Description

### CROSS-REFERENCE TO RELATED APPLICATIONS

This application claims priority to prior filed U.S. Provisional Patent Application No. 63/383,445 filed on November 11, 2022 which is hereby incorporated by reference as set forth in full herein.

### FIELD

The present invention relates generally to catheter-based medical devices, and particularly to catheters with multi-electrode end effectors.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia can include mapping the electrical properties of the endocardium and the heart volume and selectively ablating cardiac tissue by application of electrical energy, typically in the form of radiofrequency (RF) signals, and more recently, pulsed signals to induce irreversible electroporation (IRE). Through this procedure, it is possible to cease or modify the propagation of unwanted electrical signals from one portion of the heart to another. The ablation process destroys the unwanted electrical pathways by formation of non-conducting lesions.

This procedure typically utilizes at least one catheter with a multi-electrode end effector. Such muti-electrode end effectors are configured to map and/or ablate tissue. Multi-electrode end effectors come in several geometries with typical geometries being spherical, planar, and ray shaped. Some multi-electrode end effectors include spines which provide structural support to the end effector as the end effector is manipulated during a procedure.

### SUMMARY

Examples presented herein generally include catheter end effectors having a support frame with one or more corrugated struts. Corrugation of a strut increases lateral stiffness of the strut and decreases axial bending stiffness of the strut compared to a linear, non-corrugated strut of similar thickness. Geometry of corrugations can be selected to provide conformal contact of the end effector to tissue while maintaining a desired spatial relationship between electrodes of the end effector. The geometry of corrugations that can be selected can include amplitude of undulations, wavelength of undulations, and placement of a particular geometry within the end effector. End effectors having various geometries that currently utilize a rigid or semi-rigid support frame to support electrode placement can be modified to include one or more corrugated struts according to aspects of the present invention. This disclosure further contemplates inclusion of a corrugated strut in end effector geometries yet to be developed.

An example catheter can include a corrugated strut, an electrically insulating structure disposed around at least a portion of the corrugated strut, and one or more electrodes coupled to the electrically insulating structure. The corrugated strut can extend along a longitudinal axis and can have undulations with an amplitude in a first orthogonal axis orthogonal to the longitudinal axis. The electrically insulating structure can have a width greater than the amplitude of the undulations. The width is measured along a second orthogonal axis orthogonal to the longitudinal axis and orthogonal to the first orthogonal axis.

The catheter can further include plurality of corrugated struts and an array of electrodes. The struts can each extend along the longitudinal axis and can each have undulations in the first orthogonal axis. The one or more electrically insulating structures can be disposed around the plurality of corrugated struts. The array of electrodes can be disposed on the one or more electrically insulating structures. The array of electrodes can be arranged in a plane orthogonal to the first orthogonal axis.

The amplitude of the undulations can define a gap along the first orthogonal axis such that the one or more electrodes are primarily disposed outside of the gap. The gap can be defined as a column extending between a peak and a trough of the undulations. An electrode encircling the spine, therefore, is dimensioned greater than the amplitude of the undulations along the first orthogonal axis.

Wavelength of the undulations can vary along a length of the corrugated strut.

The catheter can further include a shaft and an end effector. The shaft can extend along the longitudinal axis. The end effector can be disposed at a distal end of the shaft. The end effector can include the corrugated strut, the electrically insulating structure, and the one or more electrodes. The end effector can have a planar configuration, a multi-ray configuration, a basket configuration, or a lasso configuration.

The catheter can further include a linear spine including the corrugated strut, the electrically insulating structure, and the one or more electrodes. The insulating structure of the linear spine can include an elongated member with a lumen therethrough such that at least a portion of the corrugated strut extends through the lumen. The width of the elongated member through which the corrugated strut extends can be greater than the amplitude of the undulations of the portion of the corrugated strut within the lumen. The elongated member can have a circular outer surface.

The one or more electrodes can include a ring electrode encircling the corrugated strut. The ring electrode can have an inner diameter greater than the amplitude of the undulations.

The insulating structure can include a pair of planar substrates orthogonal to the first orthogonal axis such that the corrugated strut is positioned between the pair of planar substrates. The one or more electrodes can include a pair of electrodes positioned opposite each other on the pair of planar substrates on opposite sides of the corrugated strut.

An example end effector of a catheter can include a plurality of loop members and a first support frame. The plurality of loop members can be arranged in a planar configuration such that a longitudinal axis of the catheter is parallel to a plane of the end effector, a first orthogonal axis is orthogonal to the plane of the end effector, and a second orthogonal axis is orthogonal to the longitudinal axis and orthogonal to the first orthogonal axis. The first support frame can include a first corrugated strut extending through a first loop member of the plurality of loop members.

The end effector can further include a second support frame including a second corrugated strut extending through a second loop member of the plurality of loop members. The first corrugated strut can be parallel to the second corrugated strut. The first corrugated strut and the second corrugated strut can extend parallel to the longitudinal axis.

The plurality of loop members can include outer spines parallel to the longitudinal axis. The first corrugated strut and the second corrugated strut can be respectively positioned within the outer spines. The plurality of loop members can include inner spines parallel to the longitudinal axis. As an alternative to the first and second corrugated struts being positioned within the outer spines, the first corrugated strut and the second corrugated strut can be respectively positioned within the inner spines. The end effector can include corrugated struts through the outer spines and also the inner spines or some sub-combination thereof.

The plurality of loop members can include the first loop member, a second loop member, and a third loop member. The first loop member and the third loop member can each respectively include an outer spine and an inner spine. The second loop member can include central spines each between an outer spine and an inners spine of the first and second loop members.

The end effector can further include a second support frame and a third support frame. The second support frame can extend through the second loop member. The third support frame can extend through the third loop member. The first support frame can be corrugated through a majority of a length of the first loop member. The third support frame can be corrugated through a majority of a length of the third loop member. The second support frame can be non-corrugated.

The first corrugated strut can be positioned on a distal curved portion of the first loop member.

The first loop member can include a first tubular housing at least partially surrounding the first support frame. The tubular housing can have a circular outer surface.

The first tubular housing can have a width greater than an amplitude of undulations of the first corrugated strut. The width can be measured along the second orthogonal axis. The amplitude of the undulations can be measured along the first orthogonal axis.

The end effector can further include one or more electrodes disposed on the first tubular housing. The one or more electrodes can include a plurality of electrodes linearly arranged. The plurality of electrodes can be in the plane of the end effector.

The one or more electrodes can include a ring electrode encircling the corrugated strut. The ring electrode can have an inner diameter greater than an amplitude of undulations of the first corrugated strut. The amplitude can be measured along the first orthogonal axis

Wavelength of undulations of the first corrugated strut can vary along a length of the first corrugated strut.

Another example end effector of a catheter can include an array of electrodes, and a plurality of elongated struts. The array of electrodes can be arranged in a plane. The plane can extend along a longitudinal axis of the catheter and orthogonal to a first orthogonal axis. The plurality of corrugated struts can each extend along the longitudinal axis. The struts can each be configured to maintain spatial arrangement of the array of electrodes.

The end effector can further include a plurality of spines extending along the longitudinal axis. The plurality of corrugated struts can extend through the plurality of spines. The array of electrodes can be disposed on the plurality of spines.

The end effector can further include a first membrane supported by the one or more struts on a first side of the end effector to define a generally planar first surface. The array of electrodes can be affixed to the first surface. The end effector can further include a second membrane supported by the one or more struts on a second side of the end effector to define a generally planar second surface parallel to the first surface.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
Figure 1 is an illustration of a catheter having an end effector at a distal portion of the catheter and a proximal handle at a proximal portion of the catheter according to aspects of the present invention.
Figure 2A is an illustration of an isometric view of support frames of an end effector according to aspects of the present invention.
Figure 2B is an illustration of a planar view of the support frame illustrated in Figure 2A.
Figure 3A is an illustration of another support frame of an end effector according to aspects of the present invention.
Figure 3B is an illustration of another support frame of an end effector according to aspects of the present invention.
Figure 4A is an illustration of a portion of a spine according to aspects of the present invention.
Figure 4B is an illustration of a cross-section of the spine as indicated in Figure 4A according to aspects of the present invention.
Figure 4C is an illustration of a cross-section of the spine as indicated in Figure 4A according to aspects of the present invention.
Figure 5A is an illustration of a cross-section of a spine having a wavelength that varies along a length of the spine according to aspects of the present invention.
Figure 5B is an illustration of a cross-section of a spine having a variable amplitude that varies along a length of the spine according to aspects of the present invention.
Figure 5C is an illustration of a cross-section of a spine having a variable thickness that varies along a length of the spine according to aspects of the present invention.
Figure 5D is an illustration of corrugated wave shapes according to aspects of the present invention.
Figures 6A, 6B, 6C, and 6D are illustrations of deformation of an example support frame as a result of application of various forces according to aspects of the present invention.
Figure 7A is an illustration of another example end effector according to aspects of the present invention.
Figure 7B is an illustration of a cross-section of the end effector as indicated in Figure 7A according to aspects of the present invention.
Figure 8 is an illustration of another example end effector having a ray shape according to aspects of the present invention.
Figure 9 is an illustration of another example end effector having a basket shape according to aspects of the present invention.
Figure 10 is an illustration of another example end effector having a circular or lasso shape according to aspects of the present invention.
Figure 11 is an illustration of an example catheter-based electrophysiology mapping and ablation system according to aspects of the present invention.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%.

In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator whereas "distal" indicates a location further away to the operator or physician.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the pertinent art in view of the teachings herein. Such modifications and variations are intended to be included within the scope of the claims.

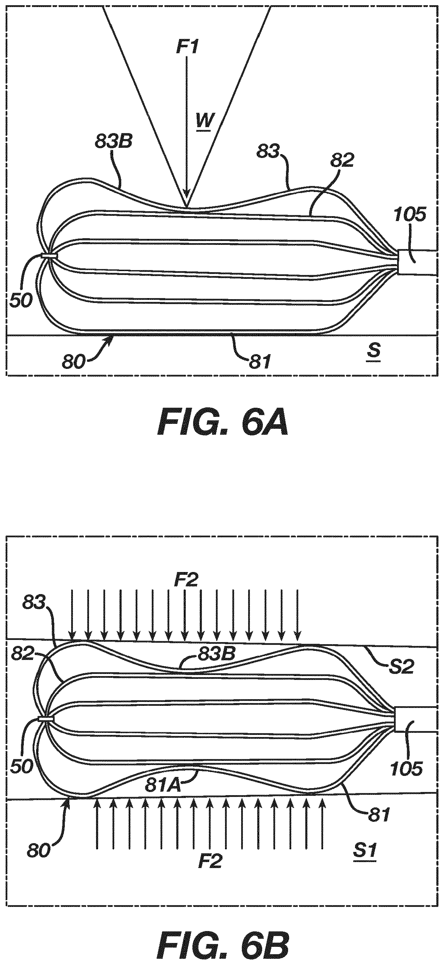

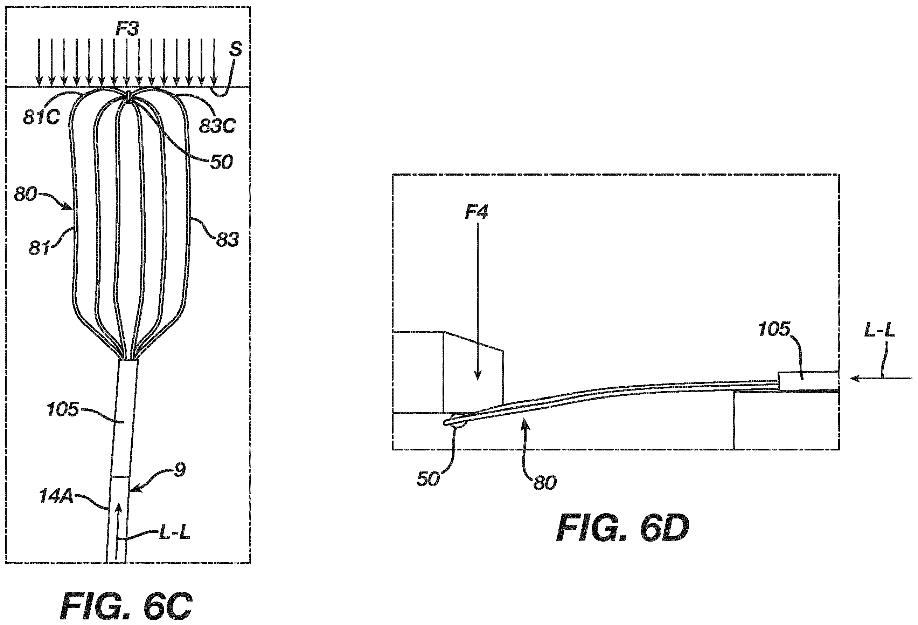

Multi-electrode end effectors can include a rigid or semi-rigid support frame to support electrode placement. The support frame can include nitinol or other bio-compatible material, and insulative materials, electrodes, conductors to electrodes, and other end-effector components can be mounted over the support frame. The support frame is configured to maintain spatial relationship between the electrodes as the end effector is manipulated and opposed to tissue. For certain catheter geometries, particularly planar end effector geometries, mechanical design requirements can be conflicting, resulting in design compromises. For instance, between the loading conditions illustrated in Figures 6A through 6D, it is desirable to have increased lateral stiffness to resist deformation under loading conditions illustrated in Figures 6A and 6B, while having minimize axial stiffness to allow for flexing under the loading condition illustrated in Figure 6D, and also while maintaining bending stiffness to resist and allow deformation within a range of loading conditions as illustrated in Figure 6C.

In examples presented herein, one or more struts of a support frame of an end effector can be corrugated. Corrugation of a strut increases lateral stiffness of the strut and decreases axial bending stiffness of the strut compared to a linear, non-corrugated strut of similar thickness. Geometry of corrugations can be selected to provide conformal contact of the end effector to tissue while maintaining a desired spatial relationship between electrodes of the end effector. The geometry that can be selected can include amplitude of undulations, wavelength of undulations, and placement of a particular geometry within the end effector. While corrugated struts may be particularly advantageous for planar end effectors, corrugated struts can also be used in numerous other end effector geometries to achieve desired mechanical functionality of an end effector support frame. The corrugations may be particularly advantageous in achieving improved conformal tissue contact, and therefore higher mapping detail in trabeculated and pectinate muscle structures.

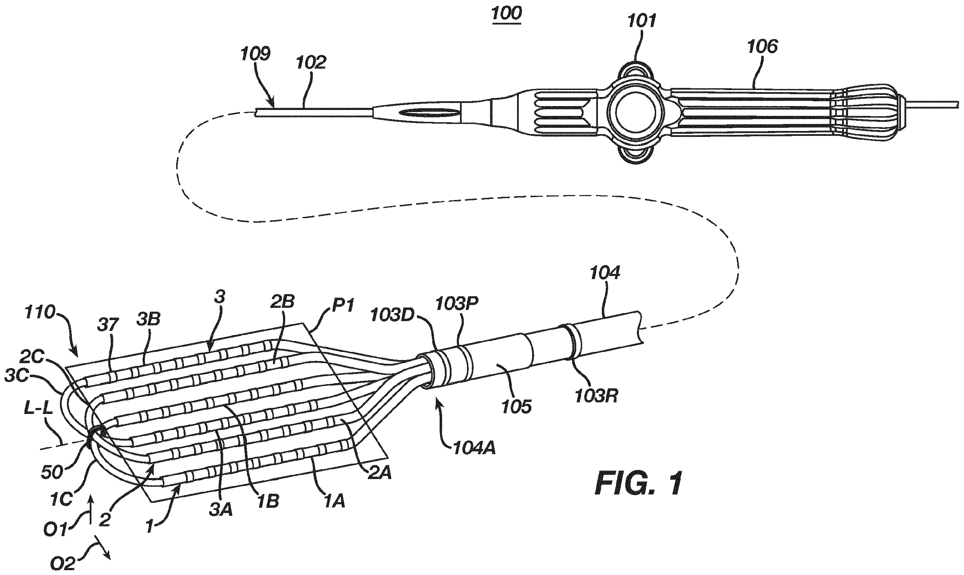

Figure 1 is an illustration of a catheter 100 including an end effector 110 at a distal portion of the catheter 100, a proximal handle 106 at a proximal portion of the catheter 100, and a shaft 109 extending between the end effector 110 and the handle 106. The elongated shaft 109 has a proximal portion 102 in the shape of an elongated catheter body, an intermediate deflection section 104, and distal portion 104A. The deflection control handle 106 is attached to the proximal end of the catheter body 102. The distal portion 104A of the shaft 109 is coupled to the end effector 110 via a connector tubing 105. The elongated shaft 109 forms a tubular catheter body sized and otherwise configured to traverse vasculature. The end effector 110 has a plurality of loop members 1, 2, 3 that overlap at a common distal vertex 50 and are joined at the distal vertex with a mechanical linkage.

When the device is unconstrained and aligned, the proximal portion 102, intermediate section 104, distal portion 104A, and end effector 110 are generally aligned along a longitudinal axis L-L. The intermediate section 104 can be configured to bend to deflect the distal portion 104A and end effector 110 from the longitudinal axis L-L.

The end effector 110 can be collapsed (compressed toward the longitudinal axis L-L) to fit within a guiding sheath or catheter (not illustrated). The shaft 109 can be pushed distally to move the end effector 110 distally through the guiding sheath. The end effector 110 can be moved to exit a distal end of the guiding sheath via manipulation of the shaft 109 and/or control handle 106. An example of a suitable guiding sheath for this purpose is the Preface Braided Guiding Sheath, commercially available from Biosense Webster, Inc. (Irvine, California, USA).

The end effector 110 has first, second and third loop members 1, 2, and 3. Each loop member 1, 2, 3 has two spines 1A, 1B, 2A, 2B, 3A, 3B and a connector 1C, 2C, 3C that connects the two spines of the respective loop member 1, 2, 3. Spines 1A, 1B of the first loop member 1 are connected by a first connector 1C; spines 2A, 2B of the second loop member 2 are connected by a second connector 2C; and spines 3A, 3B of the third loop member 3 are connected by a third connector 3C. The connectors 1C, 2C, 3C can be arcuate members as illustrated, or can have an alternative shape.

For each loop member 1, 2, 3 the spines 1A, 1B, 2A, 2B, 3A, 3B in the respective pair of spines can be substantially parallel to each other along a majority of their respective lengths when the end effector 100 is expanded in an unconstrained configuration as illustrated in Figure 1. The end effector 110 can include a support frame assembly including one or more support frames that extend through one or more of the loop members 1, 2, 3. The support frame assembly can include one or more corrugated struts through some or all of the spines 1A, 1B, 2A, 2B, 3A, 3B to provide desired lateral stiffness of the spine and decreases axial bending stiffness of the spine. Geometry of corrugations can be selected to provide conformal contact of the end effector to tissue while maintaining a desired spatial relationship between electrodes of the end effector.

Preferably, all spines in the end effector are parallel to each other along the majority of their respective lengths when the end effector 100 is in the unconstrained configuration. The end effector provides an array of electrodes 37 that are generally in a plane P1. When unconstrained, the electrodes are approximately co-planar such that when the end effector 110 is pressed to a planar surface by manipulation of the handle 106 and shaft 109, the electrodes 37 each contact the planar surface and become precisely planar. When the intermediate section 104 of the shaft 109 is non-deflected, the longitudinal axis L-L is parallel to the plane P1. A first orthogonal axis O1 is orthogonal to the plane P1 and the longitudinal axis L-L. A second orthogonal axis O2 is orthogonal to the longitudinal axis L-L and the first orthogonal axis O1. The plane P1 is parallel to the second orthogonal axis 02.

Each spine 1A, 1B, 2A, 2B, 3A, 3B can have a length ranging between about 5 and 50 mm, preferably about 10 and 35 mm, and more preferably about 28 mm. The parallel portions of each spine 1A, 1B, 2A, 2B, 3A, 3B can be spaced apart from each other by a distance ranging between about 1mm and 20 mm, preferably about 2 and 10 mm, and more preferably about 4 mm. Alternatively, parallel portions of at least some of the spines can be touching, individual spines can be adhered together, or multiple struts can extend through a single spine. Each spine 1A, 1A, 1B, 2A, 2B, 3A, 3B preferably carries at least eight electrodes per spine member. The end effector preferably includes six spines as illustrated. With eight electrodes on six spines, the end effector 100 includes forty-eight electrodes. The number of spines, number of electrodes, dimensions of spines, and spacing of electrodes can be varied to meet design objectives of the particular end effector design.

A distal electrode 103D and a proximal electrode 103P are positioned near the distal portion 104A of the shaft 109. The electrodes 103D and 103P can be configured to cooperate (e.g. by masking of a portion of one electrode and masking a different portion on the other electrode) to define a referential electrode (an electrode that is not in contact with tissues). One or more impedance sensing electrodes 103R can be configured to allow for location sensing via impedance location sensing technique, as described in US Patent Nos. 5,944,022; 5,983,126; and 6,445,864, of which copies are attached in the Appendix of priority patent application U.S. 63/383,445 and incorporated herein by reference. The configuration and placement of electrodes 103D, 103P, 103R on the distal portion 104 of the shaft 109 can be varied to meet the design objectives of the particular catheter design.

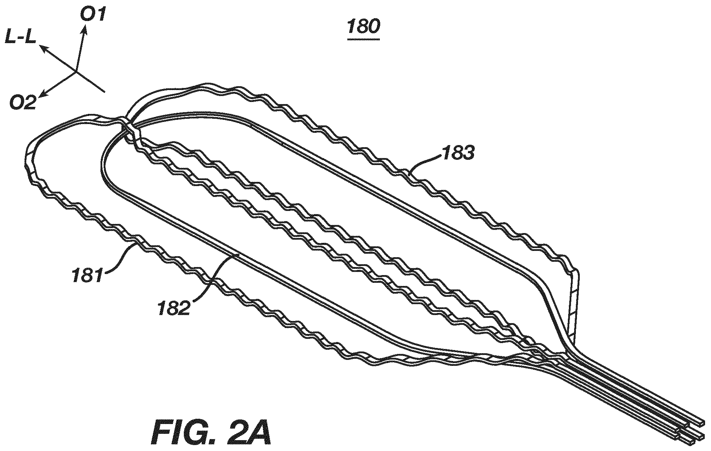

Figure 2A is an illustration of an isometric view of a support frame assembly 180 of an end effector in an unconstrained configuration. The support frame assembly 180 includes a first support frame 181, a second support frame 182, and a third support frame 183. The first support frame 181 is corrugated, and the third support frame 183 is corrugated. The second support frame 182 is not corrugated. The support frame assembly 180 can be configured to support an end effector similar to the end effector 110 illustrated in Figure 1 by modifying the loop path of the first support frame 181 and third support frame 183 or by modifying the loop path of the first loop 1 and the third loop 3 as understood by a person skilled in the pertinent art. The support frames 181, 182, 183 can include plastic or metal cut-off sheets, plastic or metal round wire, plastic or metal square wire, or other suitable biocompatible material. In the preferred embodiments, the support frames are made from shape memory material such as, for example, nitinol.

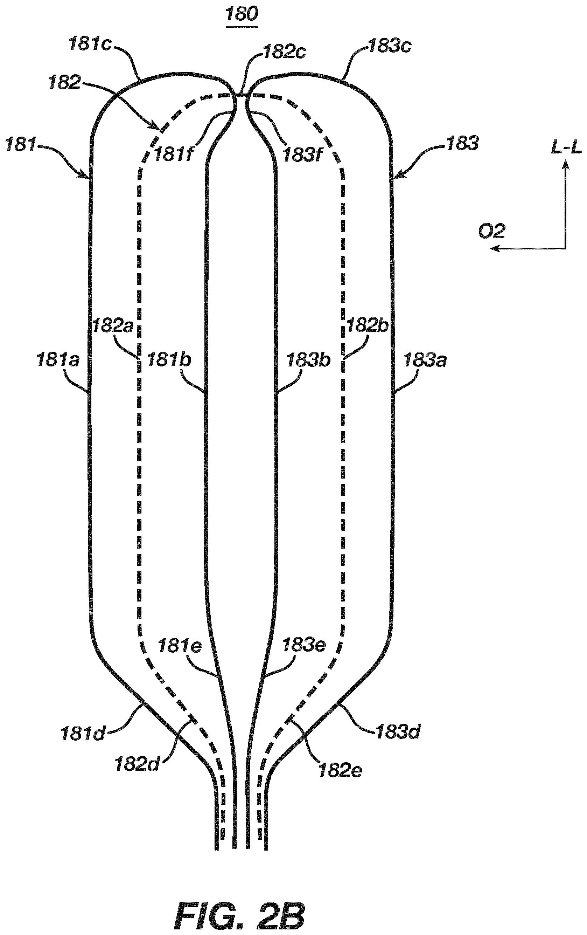

Figure 2B is an illustration of a planar view of the support frame assembly 180 illustrated in Figure 2A. When the end effector is unconstrained, each of the respective support frames 181, 182, 183 defines a respective looped path of its respective loop member (e.g. similar to loop members 1, 2, 3 illustrated in Figure 1). Each support frame 181, 182, 183 includes respective parallel segments 181a, 182a, 183a, 181b, 182b, 183b that extend through corresponding spines (e.g. similar to spines 1A, 2A, 3A, 1B, 2B, 3B illustrated in Figure 1) of the end effector 100. Each support frame 181, 182, 183 includes respective proximal segments 181d, 182d, 183d, 181e, 182e, 183e that extend through corresponding proximal segments of respective loop members. The proximal segments 181d, 182d, 183d, 181e, 182e, 183e extend into the connector tubing 105 to join the end effector 110 to the shaft 109. Each support frame 181, 182, 183 includes a respective connecting segments 181c, 182c, 183c that extends between the respective pair of parallel segments 181a, 182a, 183a, 181b, 182b, 183b and through the respective connector of the respective loop member (e.g. similar to connectors 1C, 2C, 3C illustrated in Figure 1).

The connecting segments 181c, 183c of the first support frame 181 and the third support frame 183 can each include a respective bend 181f, 183f near the apex of the connecting segment 182c of the second support frame 182 so that the connecting segments 181c, 183c of the first support frame 181 and the third support frame 183 are non-overlapping with each other at the distal vertex of the support frame assembly 180.

The support frame assembly 180 illustrated in Figures 2A and 2B includes four corrugated struts 181a, 181b, 183b, 183a. The corrugated struts are parallel to each other and approximately planar. Alternatively, any combination of the six struts 181a, 182a, 181b, 183b, 182a, 183a of the support frame assembly 180 can be corrugated. Preferably, at least the outer struts 181a, 183a are corrugated to provide increased lateral stiffness to resist deformation of the end effector under load conditions illustrated in Figures 6A and 6B. The inner spine 181b of the first support frame 181, the inner spine 183b of the third support frame 183, the central spines 182a, 182b of the second support frame 182, and any combination therefore, can be corrugated to reduce axial stiffness to allow for bending under the loading condition illustrated in Figure 6D.

While the corrugations are shown along an entire length of the corrugated struts 181a, 181b, 183b, 183a, a portion or portions of the corrugated struts 181a, 181b, 183b, 183a can be non-corrugated to provide for desired mechanical functionality of the support frame assembly 180. Further, while corrugations are shown as uniform along an entire length of the corrugated struts 181a, 181b, 183b, 183a, geometry of the corrugations such as amplitude and/or wavelength can vary along the length of the corrugated struts 181a, 181b, 183b, 183a to provide for desired mechanical functionality of the support frame assembly 180.

The connecting segments 181c, 182c, 183c can be corrugated. As illustrated, the connecting segment 181c of the first support frame 181 and the connecting segment 183c of the third support frame 183 are corrugated. Corrugation of these connecting segments 181c, 183c can increase resistance to deformation under loading conditions illustrated in Figure 6A and/or Figure 6B. Corrugation of any of the connecting segments 181c, 182c, 183c can be utilized to achieve desired deformation of the support frame assembly 180 under the loading condition illustrated in Figure 6C.

While the support frame assembly 180 is illustrated with support frames having generally uniform width and thickness along the looped path of the respective support frame 181, 182, 183, the cross-section of the support frames can vary along the looped path to provide desired mechanical functionality of the support frame assembly 180. For instance, the cross-sectional area of a support frame can be varied similar to as disclosed in U.S. Patent Publication No. 2021/0369339, incorporated herein by reference and attached in the Appendix of priority patent application U.S. 63/383,445.

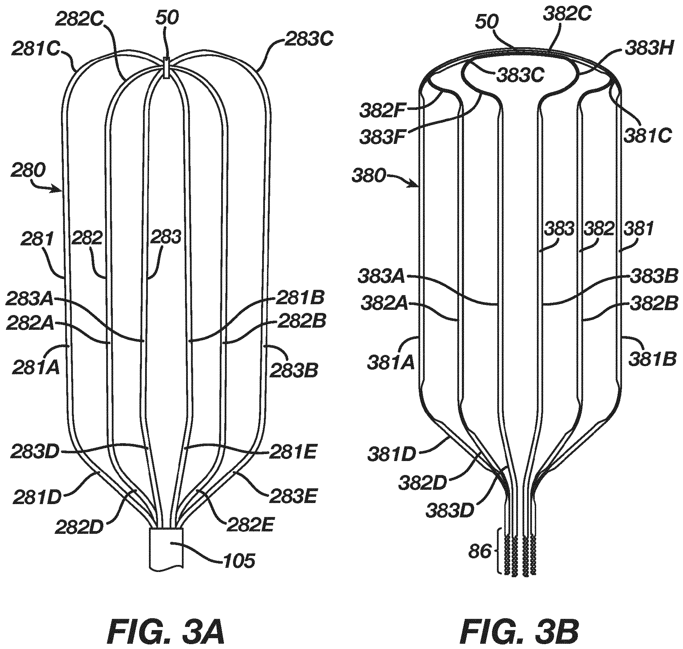

Figure 3A and 3B are illustrations of alternative planar end effector support frame assembly configurations. Numerous alternative planar end effector geometries can include corrugations as understood by a person skilled in the pertinent art according to the present disclosure.

Figure 3A is an illustration of an alternative support frame 280 of an end effector having looped paths similar to the end effector 110 illustrated in Figure 1. A first support frame 218 includes a first support frame 281, second support frame 282, and third support frame 283. Each support frame 281, 282, 283 includes respective parallel segments 281A, 282A, 283A, 281B, 282B, 283B that extend through corresponding spines 1A, 2A, 3A, 1B, 2B, 3B of the end effector 110. Each support frame 281, 282, 283 includes respective proximal segments 281D, 282D, 283D, 281E, 282E, 283E that extend through corresponding proximal segments of respective loop members 1, 2, 3. The proximal segments 281D, 282D, 283D, 281E, 282E, 283E extend into the connector tubing 105 to join the end effector 110 to the shaft 109. Each support frame 281, 282, 283 includes a respective connecting segments 281C, 282C, 283C that extends between the respective pair of parallel segments 281A, 282A, 283A, 281B, 282B, 283B and through the respective connecting segment 1C, 2C, 3C of the respective loop member 1, 2, 3.

The parallel segments 281A, 282A, 283A, 281B, 282B, 283B can be approximately coplanar when unconstrained and become precisely coplanar when the proximal portion 102 of the shaft 109 is manipulated to press the end effector 110 to a planar surface.

Portions of any of the support frames 281, 282, 283 can be corrugated to achieve desired mechanical functionality.

Figure 3B is an illustration of another support frame assembly 380 of an end effector including a first support frame 381, a second support frame 382, and the third support frame 383. The support frames 381, 382, 383 overlap at a common distal vertex 50. The support frame assembly 380 includes proximal segments 381D, 382D, 383D and parallel segments 381A, 381B, 382A, 382B, 383A, 383B configured similarly to proximal segments and parallel segments of the example support frames 180, 280 illustrated in Figures 2A, 2B, and 3A.

The first support frame 381 includes a first connecting member 381C having a curved shape between distal ends of a first pair of parallel segments 381A, 381B. The second support frame 382 includes a second connecting member 382C having a pair of curvatures 382F that extend from the distal ends of the parallel segments 382A, 382B, away from the longitudinal axis L-L, turn distally, and turn toward the longitudinal axis L-L. The second connecting member 382C has a curved shaped arc that between the pair of curvatures 382F. The third support frame 383 includes a third connecting member 383C having a pair of curvatures 383F that extend from the distal ends of the parallel segments 383A, 383B, away from the longitudinal axis L-L, turn distally, and turn toward the longitudinal axis L-L. The second connecting member 383C has a curved shaped arc that between the pair of curvatures 383F.

Each of the parallel segments 381A, 381B, 382A, 382B, 383A, 383B can be approximately equal in length to each other as measured parallel to the longitudinal axis L-L. Likewise, spines of the end effector can be approximately equal in length to each other as measured parallel to the longitudinal axis L-L.

Each of the support frames 381, 382, 383 can define a respective looped path having a cross-sectional shape orthogonal to the looped path that varies along the looped path. The cross-sectional shape can have a smaller area at bends 381K, 382K. The cross-sectional shape can have a smaller area in the over a majority of the looped path through the connecting members 381C, 382C, 383C compared to a majority of the looped path through the parallel segments 381A, 381B, 382A, 382B, 383A, 383B.

The illustrated support frame assembly 380 includes three support frames 381, 382, 383. Alternatively, the support frame assembly 380 can include two support frames including an outer support frame configured similarly to the first support frame 381 and an inner support frame configured similarly to the second support frame 382, and third support frame 383.

Portions of any of the support frames 381, 382, 383 can be corrugated to achieve desired mechanical functionality.

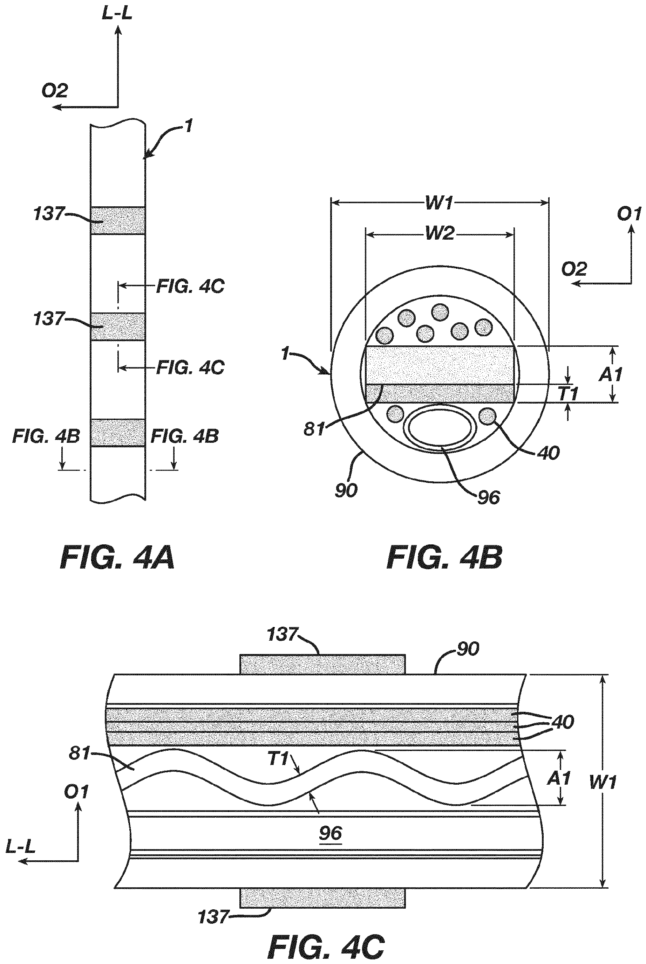

Figure 4A is an illustration of a portion of a spine 1. The spine 1 includes a portion of the end effector which carries one or more electrodes 137 and is supported by a strut 81 of a support frame. The illustrated spine 1 is linear. Alternatively, the spine 1 can be curved. As illustrated, the spine 1 is aligned linearly with the longitudinal axis L-L and the electrodes 137 on the spine 1 are aligned linearly with the longitudinal axis L-L.

Figure 4B is an illustration of a cross-section of the spine 1 as indicated in Figure 4A.

Figure 4C is an illustration of a cross-section of the spine 1 as indicated in Figure 4A.

Referring collectively to Figures 4B and 4C, spine 1 includes an elongate member 90 with a lumen therethrough, an irrigation tube 96 extending through the lumen of the elongate member 90, a corrugated strut 81 extending through the lumen of the elongate member 90, electrical conductors 40 (e.g. wires) extending through the lumen of the elongate member 90, and electrodes 137 coupled to an outer surface of the elongate member 90. The elongate member 90 is an example electrically insulating structure configured to carry the electrodes 137. The elongate member 90 as illustrated, can include an outer tube. The outer tube can have a circular cross-section as illustrated, an ovular cross-section, a rectangular cross-section, or an alternative cross-section as understood by a person skilled in the pertinent art. The electrodes 137 can have a ring shape, circumscribing the elongate member 90 as illustrated, or can be spot shaped or have other shape as understood by a person skilled in the art. The irrigation tube 96 is optional. The electrical conductors 40 can include insulated wires as illustrated, can have an alternative cross-sectional shape, can be mounted in a flex circuit, or otherwise configured as understood by a person skilled in the art.

The elongate member 90 has a width W1 measured orthogonal to the longitudinal axis L-L, along the second orthogonal axis 02. The strut 81 has a width W2 along the second orthogonal axis O2 that is less than the width W1 of the elongate member 90. The strut 81 has undulations with an amplitude A1 as measured along the first orthogonal axis O1. The amplitude A1 is less than the width W1 of the elongate member 90. The strut 81 has a thickness T1. The elongate member 90 housing the strut 81 is substantially linear over the length illustrated, while the strut undulates with multiple peaks and troughs (two periods of undulation illustrated). The elongate member 90 need not be circular as illustrated, and may be oblong, longer in the first orthogonal direction O1 compared to the second orthogonal direction O2. In which case, the amplitude A1 may be greater than the width W1 of the elongate member 90 while the elongate member 90 remains substantially liner over multiple periods of undulation of the strut. For instance, the amplitude A1 of the strut may be 1.5 times the width W1 of the elongate member 90.

The electrodes 137 can be ring shaped, circling the elongate member 90. An inner diameter of the electrodes 137 can therefore be greater than the width W2 of the strut 81 and greater than the amplitude A1 of the undulations of the strut 81. Amplitude of the undulations A1 is sufficiently small such that the path of the spine 1 (e.g. linear as illustrated) does not follow the undulations. The undulations are shaped such that the amplitude A1 defines a gap, and the electrodes are disposed entirely outside of the gap. The inner diameter of the electrodes 137 is larger than the amplitude A1 so that the strut 81 extends through the electrodes 137 without the arrangement of the electrodes 137 following the shape of the undulations. This is in contrast to an end effector support having a support frame with a wave shape having a larger amplitude than a diameter of ring electrodes thereon; such a ring electrode necessarily has at least a portion of the electrode positioned between a trough and a peak of the larger amplitude wave shape.

As an alternative, the undulations can be shaped such that the amplitude A1 defines a gap, and the spine 81 can follow the undulations to a small extent so that the electrodes 137 are disposed primarily outside the gap.

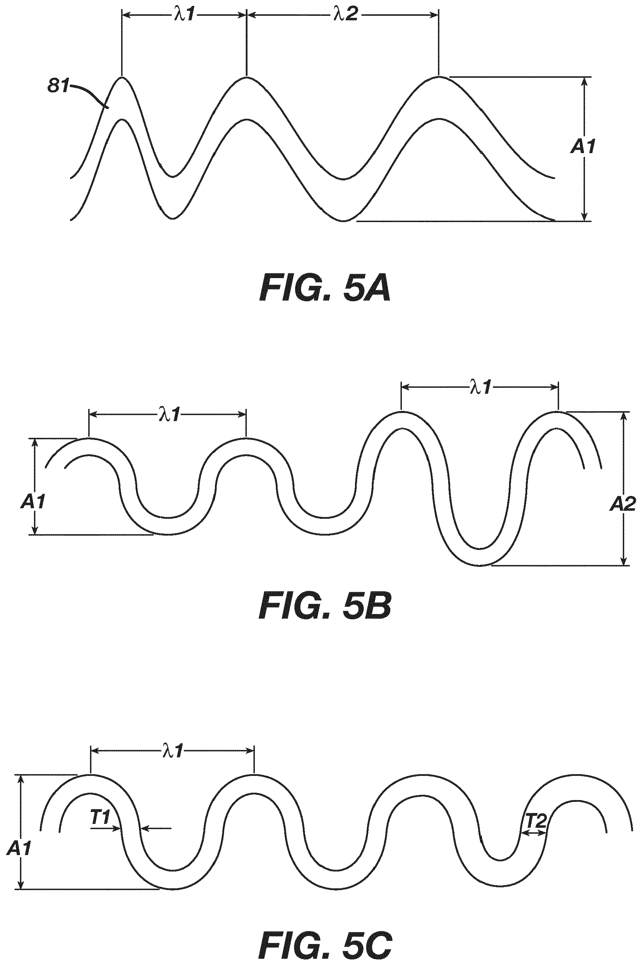

Figure 5A is an illustration of a cross-section of a spine having a variable wavelength λ₁, λ₁ that varies along a length of the spine 81. The wavelength λ₁, λ₁ can be varied to achieve the desired mechanical properties of the support frame assembly.

Figure 5B is an illustration of a cross-section of a spine having a variable amplitude A1, A2, that varies along a length of the spine 81. The amplitude A1, A2 can be varied to achieve the desired mechanical properties of the support frame assembly.

Figure 5C is an illustration of a cross-section of a spine having a variable Thickness T1, T2, that varies along a length of the spine 81. The thickness T1, T2 can be varied to achieve the desired mechanical properties of the support frame assembly.

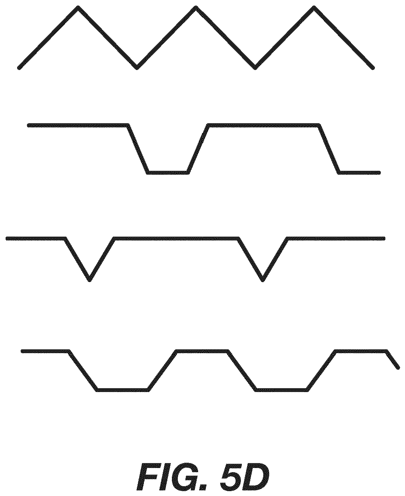

Figure 5D is an illustration of corrugated wave shapes. The undulations can be sinusoidal similar to as illustrated in Figures 4C and 5A, a half-moon similar to as illustrated in Figures 5B and 5C, triangle wave, triangle or zigzag as illustrated in Figure 5D, other corrugated shape illustrated in Figure 5D, or other corrugated wave shape as known in the art. Other dimensions of the corrugated wave shape, such as duty cycle, can be varied to achieve the desired mechanical properties of the support frame assembly. A spine 81 can have different wave shapes over different sections to achieve the desired mechanical properties of the support frame assembly.

Figures 6A, 6B, 6C, and 6D are illustrations of deformation of an example support frame assembly 80 as a result of application of various forces. The example support frame assembly 80 has a first looped support frame 81, second looped support frame 82, and third looped support frame 83

Figure 6A illustrates application of a lateral force F1 from a wedge W to an outer strut 83B of the third support frame 83 of the support frame assembly 80. An opposite outer strut of the first support frame 81 is against a planar surface S. The deformation results in the outer strut 83B of the third support frame 83 coming into contact with a strut of the second support frame 82. It may be desirable to design the support frame assembly 80 to resist this type of deformation of the support frame assembly 80 during end effector use to maintain separation between electrodes carried over the outer strut 83B and electrodes carried by the adjacent strut of the second support frame 82. Corrugating the outer strut 83B may strengthen the outer strut 83B to resist the illustrated deformation compared to a non-corrugated strut of similar thickness.

Figure 6B illustrates application of a lateral force F2 from two planar surfaces S1, S2 against outer struts 83B, 81A of the support frame assembly 80. The deformation results in the outer struts 83B, 81A coming into contact with the struts of the second support frame 82. It may be desirable to design the support frame assembly 80 to resist this type of deformation of the support frame assembly 80 during end effector use to maintain separation between electrodes carried over the outer struts 83B, 81A and electrodes carried by the respective adjacent strut of the second support frame 82. Corrugating the outer struts 83B, 81A may strengthen the outer struts 83B, 81A to resist the illustrated deformation compared to non-corrugated struts of similar thickness.

Figure 6C is an illustration of an axial force F3 from a planar surface S applied to connecting segments 81C, 83C of the support frame assembly 80. The connecting segments 81C, 83C and other portions of the support frame assembly 80 can be corrugated to provide the desired deflection as a result of the axial force F3.

Figure 6D is an illustration of a bending load force F4 applied to a distal end of the support frame assembly 80. The bending load force F4 results in deflection of the support frame assembly 80 from the longitudinal axis L-L. It may be desirable to allow deflection due to the bending load force F4 so that the end effector easily conforms to tissue when the end effector is pressed against tissue. Parallel segments and other portions of the support frame assembly 80 can be corrugated to provide the desired deflection as a result of bending load force F4.

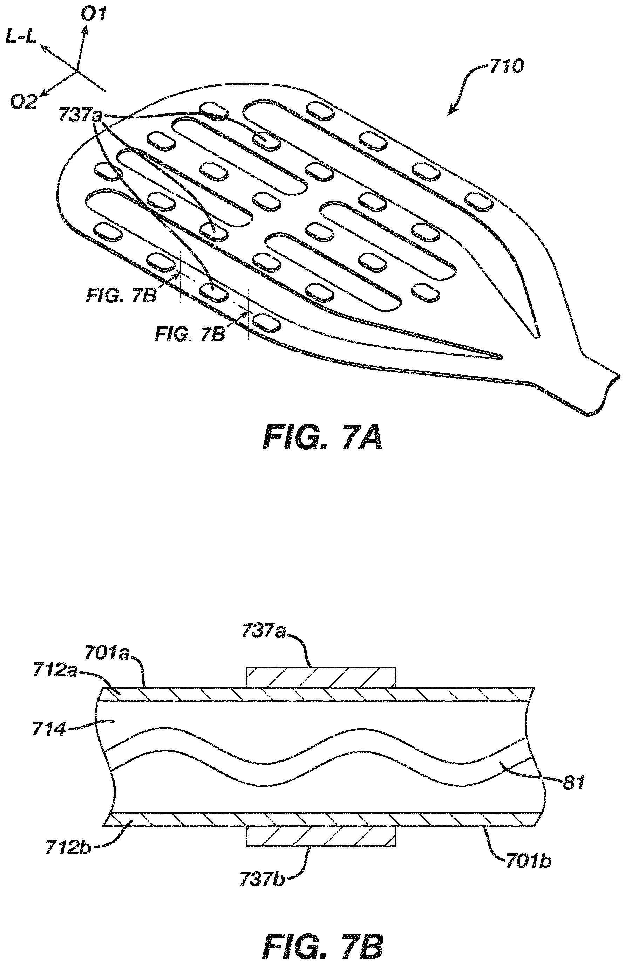

Figure 7A is an illustration of another example end effector 710 that is substantially planar.

Figure 7B is an illustration of a cross-section of the end effector 710 as indicated in Figure 7A.

Referring collectively to Figures 7A and 7B, the end effector 710 includes a first membrane 712a, first electrodes 737a, a second membrane 712b, second electrodes 737b, a corrugated strut 81, and a flexible filler 714 (e.g. polymer). The first membrane 712a can be positioned on a first side 701a of the end effector 710, and the second membrane 712b can be positioned on a second side 701b of the end effector 710. The first electrodes 737a can be coupled to the first membrane 712a exposed to ambient environment on the first surface 701a. The second electrodes 737b can be coupled to the second membrane 712b and exposed to ambient environment on the second surface 701b.

The corrugated strut 81 is positioned between the first membrane 712a and the second membrane 712b. The membranes 712a, 712b can be parallel to each other and orthogonal to the first orthogonal axis. The amplitude of the corrugated strut 81 can define a gap, and the electrodes 737a, 737b can be disposed entirely outside of the gap. At least a portion of the first electrodes 737a can be positioned opposite at least a portion of the second electrodes 737b such that one or more pairs of electrodes 737a, 737b are positioned opposite each other on the pair of planar membranes 712a, 712b on opposite sides of the corrugated strut 81.

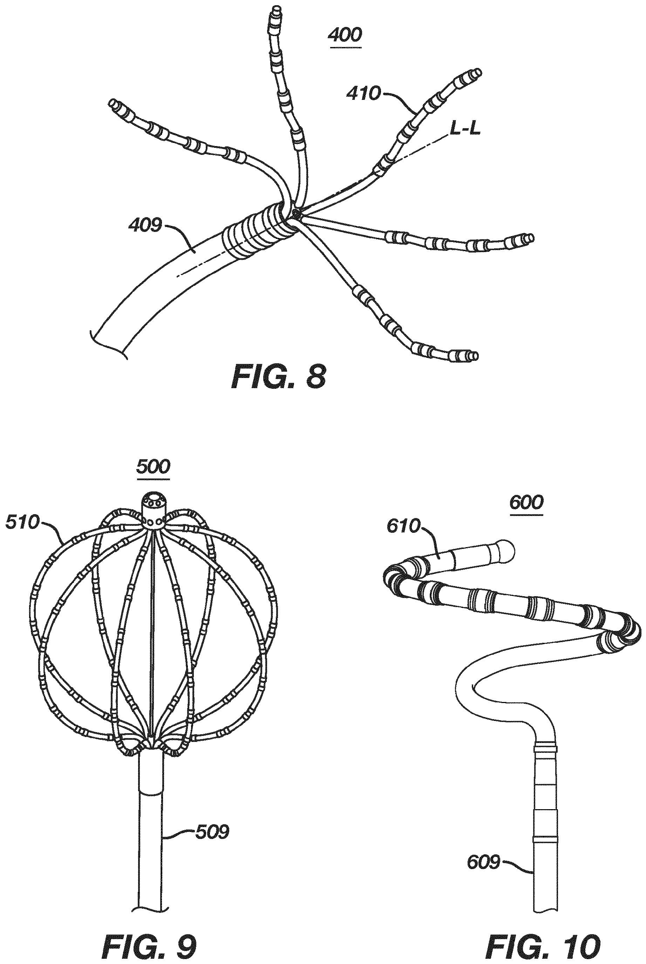

Figure 8 is an illustration of another example end effector 400 having a ray shape. Spines 410 have proximal ends that are joined to a distal end of a shaft 409 and free distal ends. One or more of the spines 410 can be configured similarly to the spine 1 illustrated in Figures 4A through 4C.

Figure 9 is an illustration of another example end effector 500 having a basket shape. Spines 510 have proximal ends that are joined to a distal end of a shaft 509 and distal ends that are joined at a hub. One or more of the spines 510 can be configured similarly to the spine 1 illustrated in Figures 4A through 4C.

Figure 10 is an illustration of another example end effector 600 having a circular or lasso shape. The end effector 600 includes a single spine 610 extending from a shaft 609. The catheter can include a pull wire extending through the spine 610 and the shaft 609 to move the spine from a straight configuration to the circular shape, and or a strut within the spine 610 can be pre-set to move the spine 610 into the lasso shape when unconstrained. The spine 610 can be configured similarly to the spine 1 illustrated in Figures 4A through 4C.

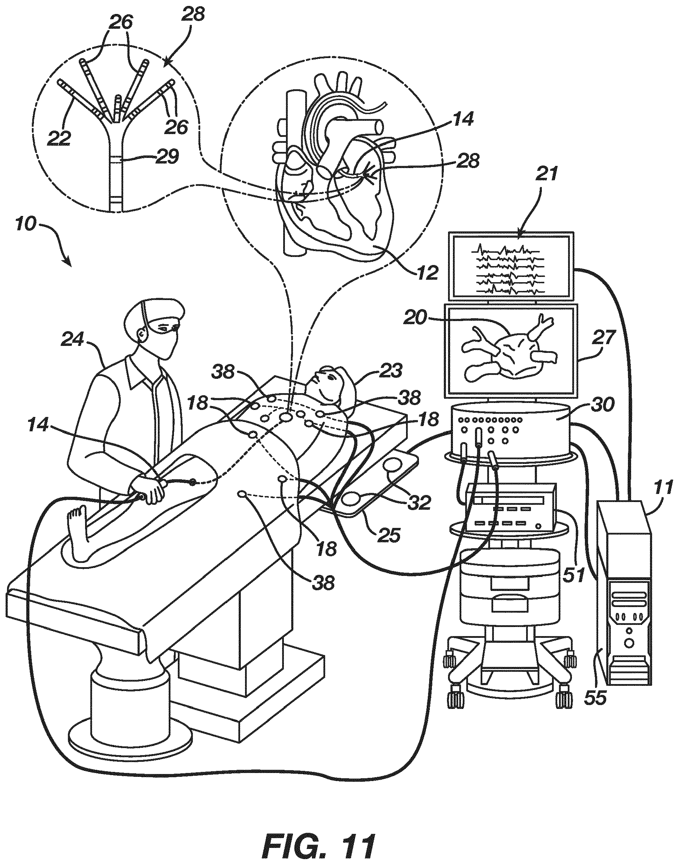

Figure 11 is an illustration showing an example catheter-based electrophysiology mapping and ablation system 10. The system 10 includes multiple catheters, which are percutaneously inserted by a physician 24 through the patient's vascular system into a chamber or vascular structure of a heart 12. Typically, a delivery sheath catheter is inserted into the left or right atrium near a desired location in the heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. The physician 24 brings a distal tip 28 of the catheter 14 into contact with the heart wall for sensing a target site in the heart 12. For ablation, the physician 24 would similarly bring a distal end of an ablation catheter to a target site for ablating.

The illustrated catheter 14 is an exemplary catheter that includes one and preferably multiple electrodes 26 optionally distributed over a plurality of spines 22 at distal tip 28 and configured to sense the IEGM signals. Catheter 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation. The catheter 14 can otherwise be configured similarly to other example catheters presented herein, variations thereof, and alternatives thereto as understood by a person skilled in the pertinent art. The distal portion 28 can include one or more corrugated struts.

A magnetic based position sensor 29 may be operated together with a location pad 25 including a plurality of magnetic coils 32 configured to generate magnetic fields in a predefined working volume. Real time position of a distal tip 28 of the catheter 14 may be tracked based on magnetic fields generated with a location pad 25 and sensed by a magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091 incorporated by reference herein and attached in the Appendix of priority patent application U.S. 63/383,445.

The system 10 includes one or more electrode patches 38 positioned for skin contact on the patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182 incorporated by reference herein and attached in the Appendix of priority patent application U.S. 63/383,445.

A recorder 11 displays electrograms 21 captured with body surface ECG electrodes 18 and intracardiac electrograms (IEGM) captured with electrodes 26 of the catheter 14. The recorder 11 may include pacing capability for pacing the heart rhythm and/or may be electrically connected to a standalone pacer.

The system 10 can include an ablation energy generator 51 that is adapted to conduct ablative energy to one or more of electrodes at a distal tip of a catheter configured for ablating. Energy produced by the ablation energy generator 51 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

A patient interface unit (PIU) 30 is an interface configured to establish electrical communication between catheters, electrophysiological equipment, power supply and a workstation 55 for controlling operation of system 10. Electrophysiological equipment of the system 10 may include for example, multiple catheters, a location pad 25, body surface ECG electrodes 18, electrode patches 38, an ablation energy generator 51, and a recorder 11. Optionally and preferably, the PIU 30 includes processing capability for implementing real-time computations of location of the catheters and for performing ECG calculations.

The workstation 55 includes memory, processor unit with memory or storage with appropriate operating software loaded therein, and user interface capability. The workstation 55 can be configured to provide multiple functions, optionally including (1) modeling the endocardial anatomy in three-dimensions (3D) and rendering the model or an anatomical map 20 for display on a display device 27; (2) displaying on the display device 27 activation sequences (or other data) compiled from recorded electrograms 21 in representative visual indicia or imagery superimposed on the rendered anatomical map 20; (3) displaying real-time location and orientation of multiple catheters within the heart chamber; and (4) displaying on the display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

The following clauses list non-limiting embodiments of the disclosure:
Clause 1. A catheter comprising: a corrugated strut extending along a longitudinal axis and comprising undulations with an amplitude in a first orthogonal axis orthogonal to the longitudinal axis; an electrically insulating structure disposed around at least a portion of the corrugated strut and comprising a width greater than the amplitude of the undulations, the width being measured along a second orthogonal axis orthogonal to the longitudinal axis and orthogonal to the first orthogonal axis; and one or more electrodes coupled to the electrically insulating structure.
Clause 2. The catheter of clause 1, further comprising: a plurality of corrugated struts, each extending along the longitudinal axis and each comprising undulations in the first orthogonal axis; one or more electrically insulating structures disposed around the plurality of corrugated struts; and an array of electrodes disposed on the one or more electrically insulating structures.
Clause 3. The catheter of clause 2, wherein the array of electrodes are arranged in a plane orthogonal to the first orthogonal axis.
Clause 4. The catheter of any one of clauses 1-3, the amplitude of the undulations defining a gap along the first orthogonal axis such that the one or more electrodes are primarily disposed outside of the gap.
Clause 5. The catheter of any one of clauses 1-4, a wavelength of the undulations varying along a length of the corrugated strut.
Clause 6. The catheter of any one of clauses 1-5, further comprising: a shaft extending along the longitudinal axis; and an end effector disposed at a distal end of the shaft, the end effector comprising the corrugated strut, the electrically insulating structure, and the one or more electrodes.
Clause 7. The catheter of clause 6, the end effector comprising a planar configuration, a multi-ray configuration, a basket configuration, or a lasso configuration.
Clause 8. The catheter of any one of clauses 1-7, further comprising: a linear spine comprising the corrugated strut, the electrically insulating structure, and the one or more electrodes.
Clause 9. The catheter of clause 8, the insulating structure of the linear spine comprising an elongated member with a lumen therethrough such that at least a portion of the corrugated strut extends through the lumen and the width of the elongated member through which the corrugated strut extends is greater than the amplitude of the undulations of the portion of the corrugated strut within the lumen.
Clause 10. The catheter of clause 9, wherein the elongated member comprises a circular outer surface.
Clause 11. The catheter of any one of clauses 1-10, the one or more electrodes comprising a ring electrode encircling the corrugated strut, the ring electrode comprising an inner diameter greater than the amplitude of the undulations.
Clause 12. The catheter of any one of clauses 1-7, the insulating structure comprising a pair of planar membranes orthogonal to the first orthogonal axis such that the corrugated strut is positioned between the pair of planar membranes.
Clause 13. The catheter of clause 12, the one or more electrodes comprising a pair of electrodes positioned opposite each other on the pair of planar membranes on opposite sides of the corrugated strut.
Clause 14. An end effector of a catheter, the end effector comprising: a plurality of loop members arranged in a planar configuration such that a longitudinal axis of the catheter is parallel to a plane of the end effector, a first orthogonal axis is orthogonal to the plane of the end effector, and a second orthogonal axis is orthogonal to the longitudinal axis and orthogonal to the first orthogonal axis; and a first support frame comprising a first corrugated strut extending through a first loop member of the plurality of loop members.
Clause 15. The end effector of clause 14 further comprising: a second support frame comprising a second corrugated strut extending through a second loop member of the plurality of loop members.
Clause 16. The end effector of clause 15, the first corrugated strut being parallel to the second corrugated strut.
Clause 17. The end effector of clause 15 or 16, the first corrugated strut and the second corrugated strut extending parallel to the longitudinal axis.
Clause 18. The end effector of any one of clauses 15-17, the plurality of loop members comprising outer spines parallel to the longitudinal axis, and the first corrugated strut and the second corrugated strut being respectively positioned within the outer spines.
Clause 19. The end effector of any one of clauses 15-17, the plurality of loop members comprising inner spines parallel to the longitudinal axis, and the first corrugated strut and the second corrugated strut being respectively positioned within the inner spines.
Clause 20. The end effector of any one of clauses 15-19, the plurality of loop members comprising the first loop member, a second loop member, and a third loop member, the first loop member and the third loop member each respectively comprising an outer spine and an inner spine, and the second loop member comprising central spines each between an outer spine and an inners spine of the first and second loop members.
Clause 21. The end effector of clause 20, further comprising: a second support frame extending through the second loop member; and a third support frame extending through the third loop member.
Clause 22. The end effector of clause 21, the first support frame being corrugated through a majority of a length of the first loop member, and the third support frame being corrugated through a majority of a length of the third loop member.
Clause 23. The end effector of clause 21 or 22, the second support frame being non-corrugated.
Clause 24. The end effector of clause 14, the first corrugated strut being positioned on a distal curved portion of the first loop member.
Clause 25. The end effector of any one of clauses 14-24, the first loop member comprising a first tubular housing at least partially surrounding the first support frame.
Clause 26. The end effector of clause 25, wherein the tubular housing comprises a circular outer surface.
Clause 27. The end effector of clause 25 or 26, the first tubular housing comprising a width greater than an amplitude of undulations of the first corrugated strut, the width being measured along the second orthogonal axis, and the amplitude being measured along the first orthogonal axis.
Clause 28. The end effector of any one of clauses 25-27, further comprising: one or more electrodes disposed on the first tubular housing.
Clause 29. The end effector of clause 28, the one or more electrodes comprising a plurality of electrodes linearly arranged.
Clause 30. The end effector of clause 29, the plurality of electrodes being in the plane of the end effector.
Clause 31. The end effector of any one of clauses 28-29, the one or more electrodes comprising a ring electrode encircling the corrugated strut.
Clause 32. The end effector of clause 31, the ring electrode comprising an inner diameter greater than an amplitude of undulations of the first corrugated strut, the amplitude being measured along the first orthogonal axis.
Clause 33. The end effector of any one of clauses 14-32, a wavelength of undulations of the first corrugated strut varying along a length of the first corrugated strut.
Clause 34. An end effector of a catheter, the end effector comprising: an array of electrodes arranged in a plane along a longitudinal axis of the catheter and orthogonal to a first orthogonal axis; and a plurality of corrugated struts each extending along the longitudinal axis and configured to maintain spatial arrangement of the array of electrodes.
Clause 35. The end effector of clause 34, further comprising: a plurality of spines extending along the longitudinal axis, the plurality of corrugated struts extending through the plurality of spines, and the array of electrodes being disposed on the plurality of spines.
Clause 36. The end effector of clause 34, further comprising: a first membrane supported by the one or more struts on a first side of the end effector to define a generally planar first surface, the array of electrodes being affixed to the first surface; and a second membrane supported by the one or more struts on a second side of the end effector to define a generally planar second surface parallel to the first surface.

Having shown and described exemplary embodiments of the subject matter contained herein, further adaptations of the methods and systems described herein may be accomplished by appropriate modifications without departing from the scope of the claims. In addition, where methods and steps described above indicate certain events occurring in certain order, it is intended that certain steps do not have to be performed in the order described but in any order as long as the steps allow the embodiments to function for their intended purposes. Therefore, to the extent there are variations of the invention, which are within the spirit of the disclosure or equivalent to the inventions found in the claims, it is the intent that this patent will cover those variations as well. Some such modifications should be apparent to those skilled in the art. For instance, the examples, embodiments, geometries, materials, dimensions, ratios, steps, and the like discussed above are illustrative. Accordingly, the claims should not be limited to the specific details of structure and operation set forth in the written description and drawings.

## Claims

1. A catheter comprising:
a corrugated strut extending along a longitudinal axis and comprising undulations with an amplitude in a first orthogonal axis orthogonal to the longitudinal axis;
an electrically insulating structure disposed around at least a portion of the corrugated strut and comprising a width greater than the amplitude of the undulations, the width being measured along a second orthogonal axis orthogonal to the longitudinal axis and orthogonal to the first orthogonal axis; and
one or more electrodes coupled to the electrically insulating structure.

2. The catheter of claim 1, further comprising:
a plurality of corrugated struts, each extending along the longitudinal axis and each comprising undulations in the first orthogonal axis;
one or more electrically insulating structures disposed around the plurality of corrugated struts; and
an array of electrodes disposed on the one or more electrically insulating structures;
optionally wherein the array of electrodes are arranged in a plane orthogonal to the first orthogonal axis.

3. The catheter of any preceding claim, the amplitude of the undulations defining a gap along the first orthogonal axis such that the one or more electrodes are primarily disposed outside of the gap.

4. The catheter of any preceding claim, a wavelength of the undulations varying along a length of the corrugated strut.

5. The catheter of any preceding claim, further comprising:
a shaft extending along the longitudinal axis; and
an end effector disposed at a distal end of the shaft, the end effector comprising the corrugated strut, the electrically insulating structure, and the one or more electrodes.

6. The catheter of claim 1, further comprising:
a linear spine comprising the corrugated strut, the electrically insulating structure, and the one or more electrodes, the one or more electrodes comprising a ring electrode encircling the electrically insulating structure, and the ring electrode comprising an inner diameter greater than the amplitude of the undulations, optionally the electrically insulating structure of the linear spine comprising an elongated member with a lumen therethrough such that at least a portion of the corrugated strut extends through the lumen and the width of the elongated member through which the corrugated strut extends is greater than the amplitude of the undulations of the portion of the corrugated strut within the lumen.

7. The catheter of any preceding claim, the electrically insulating structure comprising a pair of planar membranes orthogonal to the first orthogonal axis such that the corrugated strut is positioned between the pair of planar membranes, optionally the one or more electrodes comprising a pair of electrodes positioned opposite each other on the pair of planar membranes on opposite sides of the corrugated strut.

8. An end effector of a catheter, the end effector comprising:
a plurality of loop members arranged in a planar configuration such that a longitudinal axis of the catheter is parallel to a plane of the end effector, a first orthogonal axis is orthogonal to the plane of the end effector, and a second orthogonal axis is orthogonal to the longitudinal axis and orthogonal to the first orthogonal axis; and
a first support frame comprising a first corrugated strut extending through a first loop member of the plurality of loop members.

9. The end effector of claim 8 further comprising:
a second support frame comprising a second corrugated strut extending through a second loop member of the plurality of loop members.

10. The end effector of claim 9, the first corrugated strut being parallel to the second corrugated strut and extending parallel to the longitudinal axis.

11. The end effector of any one of claims 8 to 10, the plurality of loop members comprising (i) outer spines parallel to the longitudinal axis, and the first corrugated strut and the second corrugated strut being respectively positioned within the outer spines, or (ii) inner spines parallel to the longitudinal axis, and the first corrugated strut and the second corrugated strut being respectively positioned within the inner spines.

12. The end effector of claim any one of claims 8 to 11,
the plurality of loop members comprising the first loop member, a second loop member, and a third loop member,
the first loop member and the third loop member each respectively comprising an outer spine and an inner spine, and
the second loop member comprising central spines each between an outer spine and an inners spine of the first and second loop members.

13. The end effector of claim 8, the first corrugated strut being positioned on a distal curved portion of the first loop member.

14. The end effector of any one of claims 8 to 13, the first loop member comprising a first tubular housing at least partially surrounding the first support frame, the first tubular housing comprising a width greater than an amplitude of undulations of the first corrugated strut, the width being measured along the second orthogonal axis, and the amplitude being measured along the first orthogonal axis.

15. The end effector of claim 14, the first loop member comprising a tubular housing at least partially surrounding the first support frame, the end effector further comprising:
one or more electrodes disposed on the tubular housing and linearly arranged in the plane of the end effector, the one or more electrodes comprising a ring electrode encircling the tubular housing, and the ring electrode comprising an inner diameter greater than an amplitude of undulations of the first corrugated strut, the amplitude being measured along the first orthogonal axis.

16. The end effector of claim 8, a wavelength of undulations of the first corrugated strut varying along a length of the first corrugated strut.
